# EUROPEAN PATENT APPLICATION

(11) **EP 2 423 177 A1**
(43) Date of publication of application: **29.02.2012**
(21) Application number: 10174055.3
(22) Date of filing: 25.08.2010
(51) Int. Cl.: C07C 45/71, C07C 49/203, C07C 49/217, C07C 49/227, C07C 251/36, C07C 251/38, C07C 317/08, A61K 31/10, A61K 31/121, A61K 31/15

(54) **Analogs of geranylgeranylacetone (GGA)**

(71) Applicant: Nyken Holding B.V., 9713 GX Groningen (NL)
(72) Inventor: The designation of the inventor has not yet been filed
(74) Representative: Jansen, Cornelis Marinus

(57) **Abstract**

The invention relates to novel therapeutic compounds, more in particular to biologically active analogs of geranylgeranylacetone (GGA), and various use thereof. Provided is a compound of the general formula I

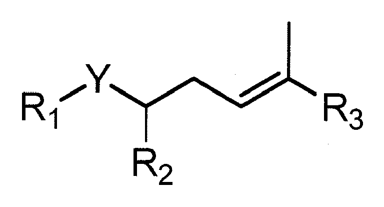

## Description

The invention relates to novel therapeutic compounds, more in particular to biologically active analogs of geranylgeranylacetone (GGA), uses of the analogs and to methods for providing them.

GGA is an acyclic polyisoprenoid that has been used to protect gastric mucosa. GGA has been shown to activate transcription factors, particularly heat shock transcription factor (HSF)-1, which are able to bind to DNA and induce transcription. HSF-1 is normally suppressed since it is typically bound to the C-domain of constitutively active HSP70. GGA is able to bind to the C-domain of the HSP70 thereby causing HSF-1 to dissociate. HSF-1 is now able to undergo trimerization and be translocated to the nucleus, where it binds to the heat shock-responsive element (HSE) in the promoter region of inducible HSP70 (i.e. HSP72) genes.

Since the 1980s GGA, also known as teprenone, is marketed as an anti-ulcer drug in Japan under the commercial name Selbex. It is widely used and appears to be non-toxic. Recent investigation revealed novel potential of this agent as a general cytoprotective compound. For example, GGA eased ischemic brain injury, endotoxin shock, atrial fibrillation, showed antiinflammatory potential and could procure potential therapeutic advantages in the prevention and treatment of ischemialreperfusion disorders, injury, inflammation, infection and organ transplants. Several patents claim the use of GGA in the treatment of glaucoma, hepatitis C, dermatosis or as a topical anti-aging active substance. In the majority of cases the effect of GGA is ascribed to its heat shock protein inducing ability. Whereas especially the relationship between GGA and the 70-kDa heat shock protein HSP72 is well-established, effects mediated by small heat shock proteins such as HSP27 are also reported. For example, W02006062402 discloses that GGA can increase the amount of HSP27 or a HSP27- like protein in a cardiac cell, providing for the use of GGA for manufacture of a medicament for the treatment of a supraventricular arrhythmia e.g., atrial fibrillation.

The chemical structure of geranylgeranylacetone (IUPAC name: (5E,9E,13E)-6,10,14,18-tetramethylnonadeca-5,9,13,17-tetraen-2-one) is as follows:

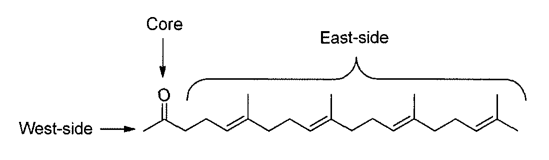

GGA is compromised of three parts: a 17 carbon long alkyl chain on the east-side, a carbonyl fragment (ketone) at its core and a west-side terminal methyl group. The hydrophobic, apolar alkyl chain on the east-side consists of four repeating isopropenoid fragments. The trivial name of two such fragments is "geranyl". Three of such consecutive fragments are trivially called "farnesyl" and four isopropenoids are referred to as "geranylgeranyl". The core is a slightly polarized C=O bond with a small positive charge on the carbon and a small negative charge on the oxygen. The oxygen of ketones can act as a hydrogen bond acceptor.

One of the disadvantages of the utilization of GGA for therapeutic purposes is the relatively high amounts needed in order to be effective. The

In one aspect, the invention provides a compound of the general formula I 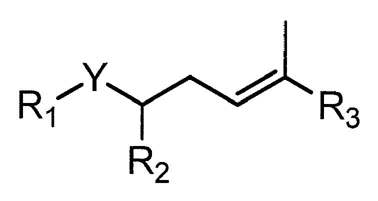 wherein
Y is selected from the group consisting of 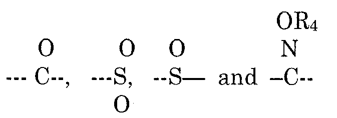 wherein R4 is hydrogen or a lower (e.g. C1-C4) alkyl group
R1 is a lower alkyl group substituted with 1 to 4 substituents selected from the group consisting of halogen, hydroxyl; lower alkyl; lower alkoxy; halogenated lower alkyl; halogenated lower alkoxy; cyano; a 5- or 6-membered (hetero)aromatic ring which may be substituted by hydroxyl, lower alkyl, lower alkoxy, halogen, amino, lower alkylamino; cyano, nitro; and other (substituted) (hetero)aromatic rings;
R2 is hydrogen, lower alkyl or substituted lower (e.g. C1-C5) alkyl group; H or methyl is preferred. Both the R and S configurations are encompassed.
R3 is a saturated or unsaturated aliphatic moiety of 1-15 carbon atoms, preferably C1-C10, optionally substituted with 1 to 5 substituents selected from the group consisting of halogen, hydroxyl, lower alkyl, lower alkoxy, hydroxyl-lower alkyl, halogen, amino, lower alkylamino and cyano, nitro, a 5-or 6-membered hetero)aromatic ring which may be substituted by hydroxyl, lower alkyl, lower alkoxy, amino, lower alkylamino; cyano, nitro; and other (substituted) (hetero)aromatic rings,
or a pharmaceutically acceptable salt thereof.

It was found that the carbonyl core moiety can be changed provided that at least some hydrogen bond acceptability is maintained.
In one embodiment, Y is 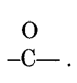 In another embodiment Y is 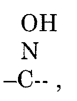 Also provided is a compound of the general formula I 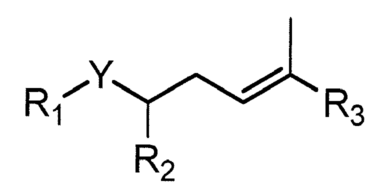 wherein
Y is selected from the group consisting of 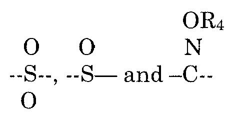 wherein R4 is hydrogen or a lower (e.g. C1-C4) alkyl group
R1 is a lower alkyl group, optionally substituted with 1 to 4 substituents selected from the group consisting of halogen, hydroxyl; lower alkyl; lower alkoxy; halogenated lower alkyl; halogenated lower alkoxy; cyano; a 5- or 6-membered (hetero)aromatic ring which may be substituted by hydroxyl, lower alkyl, lower alkoxy, amino, lower alkylamino; cyano, nitro; and other (substituted) (hetero)aromatic rings;
R2 is hydrogen, lower alkyl or substituted lower (e.g. Cl-C5) alkyl group; H or methyl is preferred. Both R and S configurations are encompassed.
R3 is a saturated or unsaturated aliphatic moiety of 1-15 carbon atoms, preferably Cl-C10, optionally substituted with 1 to 5 substituents selected from the group consisting of halogen, hydroxyl, lower alkyl, lower alkoxy, hydroxyl-lower alkyl, halogen, amino, lower alkylamino and cyano, nitro, a 5-or 6-membered hetero)aromatic ring which may be substituted by hydroxyl, lower alkyl, lower alkoxy, halogen, amino, lower alkylamino; cyano, nitro; and other (substituted) (hetero)aromatic rings,
or a pharmaceutically acceptable salt thereof.
As used herein, an alkyl group is a straight chained or branched non-aromatic hydrocarbon which is completely saturated. Typically, a straight chained or branched alkyl group has from 1 to about 20 carbon atoms, preferably from 1 to about 10, and a cyclic alkyl group has from 3 to about 10 carbon atoms, preferably from 3 to about 8. Examples of straight chained and branched alkyl groups include methyl, ethyl, n-propyl, iso- propyl, n-butyl, sec-butyl, tert-butyl, pentyl, hexyl, pentyl and octyl. Lower alkyl is a straight or branched alkyl group containing from 1 -8 carbon atoms, such as methyl, ethyl, n-propyl, iso-propyl, n-butyl, sec-butyl, tert-butyl, pentyl, hexyl, pentyl, octyl and the like.

In a compound of the invention, R1 is a lower alkyl group, optionally substituted with 1 to 4 substituents selected from the group consisting of halogen, hydroxyl; lower alkyl; lower alkoxy; halogenated lower alkyl; halogenated lower alkoxy; cyano; a 5- or 6-membered (hetero)aromatic ring which may be substituted by hydroxyl, lower alkyl, lower alkoxy, amino, lower alkylamino; cyano, nitro or other (substituted) (hetero)aromatic rings. Preferred R1 groups include lower alkyls substituted with one or more of halogen, hydroxyl, methoxy, cyano, and 5-6 membered aromatic rings, the rings being optionally substituted.

R2 is hydrogen, lower alkyl or substituted lower (e.g. C1-C5) alkyl group. In one embodiment, R2 is hydrogen. In another embodiment, it is an optionally substituted C1-C3 alkyl group. Both the R and S configuration are envisaged in case of chirality.

The east side of the molecule should have sufficient hydrophobicity. R3 is a saturated or unsaturated aliphatic moiety of 1-15 carbon atoms, preferably Cl-C10, optionally substituted with 1 to 5 substituents selected from the group consisting of halogen, hydroxyl, lower alkyl, lower alkoxy, hydroxyl-lower alkyl, halogen, amino, lower alkylamino and cyano, nitro, a 5- or 6-membered hetero)aromatic ring which may be substituted by hydroxyl, lower alkyl, lower alkoxy, halogen, amino, lower alkylamino; cyano, nitro or other (substituted) (hetero)aromatic rings. R3 is for example selected from the group consisting of substituted and unsubstituted geranylgeranyl, farnesyl and geranyl moieties. In one embodiment, R3 is an unsubstituted aliphatic moiety of 1-15, preferably 1-10 carbon atoms. In one embodiment, R3 is based on a terpene. Terpenes are derived biosynthetically from units of isoprene, which has the molecular formula C₅H₈. The basic molecular formulae of terpenes are multiples of that, (C₅H₈)n where n is the number of linked isoprene units. isoprene units may be linked together "head to tail" to form linear chains or they may be arranged to form rings. R3 may comprise or consist of 1 to 5 isoprene units, i.e. n is 1 to 5. For example, the R3 group can be (derived from) a monoterpene consisting of two isoprene units and having the molecular formula CloHi6. Examples of monoterpenes are: geraniol, limonene and terpineol. As another example, R3 is (derived from) a sesquiterpene consisting of three isoprene units and have the molecular formula C₁₅H₂₄. Examples of sesquiterpenes are: farnesenes, farnesol. In yet another example, R3 is (derived from) a diterpenes composed of four isoprene units and having the molecular formula C₂₀H₃₂.
In a specific aspect, R3 is geranylgeranyl (1), farnesyl (2) or geranyl (3) moiety.

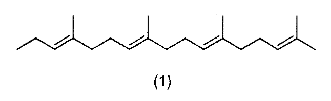

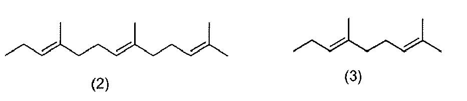

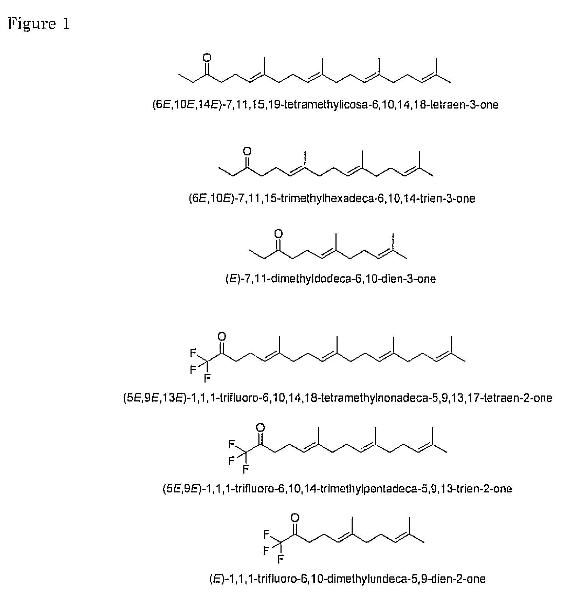

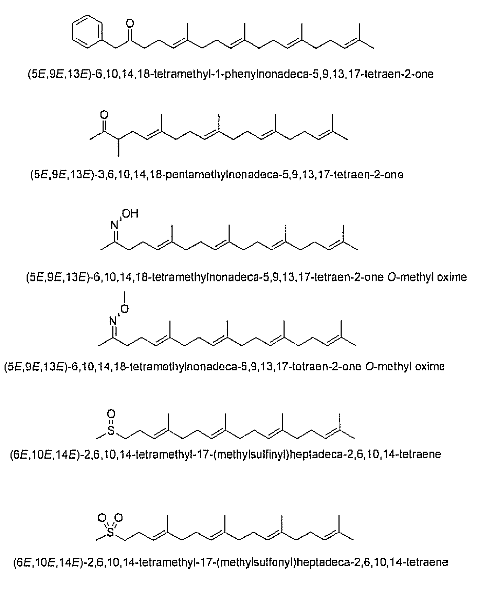

Exemplary GGA-like compounds according to the present invention are depicted in Table 1 and Figure 1.

**Table 1: Novel GGA analogs (GGA itself is shown as well).**

| | Compound |
|---|---|
| **GGA** | 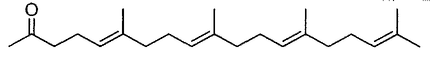 |
| **1** | 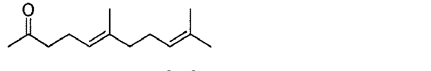 |
| **2** | 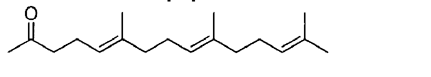 |
| **3** | 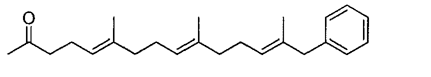 |
| **4** | 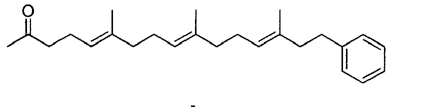 |
| **5** | 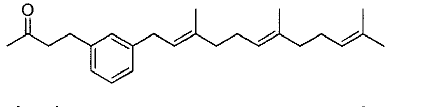 |
| **6** | 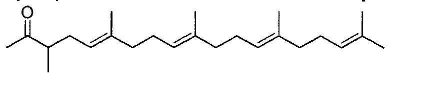 |
| **7** | 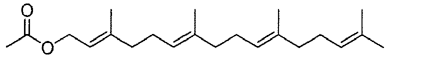 |
| **8** | 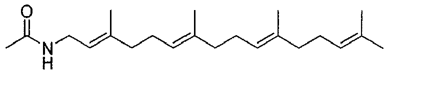 |
| **9** | 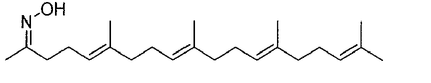 |
| **10** | 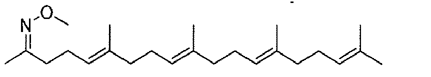 |
| **11** | 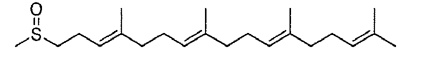 |
| **12** | 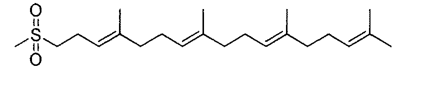 |
| **13** | 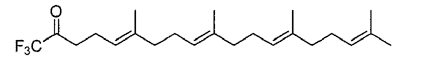 |
| **14** | 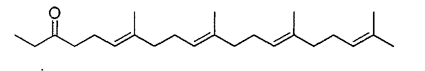 |
| **15** | 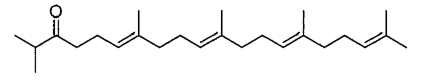 |

Preferred compounds include those listed in Table 1 with entry numbers 1-10 and 13-15. Most preferred are entries 6, 9, 10 and 13-15.

The invention also provides a method for the synthesis of a GGA-like compound of the invention. General methods known in the art can be applied using commercially available precursors. See Examples 1 to 4 for exemplary embodiments. Depending on the structure of the desired compound, the skilled person will be able to select the appropriate routes and starting materials. In one embodiment, a Carroll rearrangement of commercially available geranyl linalool (mixture of isomers) with the appropriate alkyl acetoacetate (commercially available or synthesized) is advantageously used. The Carroll rearrangement (Carroll, M. F. "131. Addition of a,8-unsaturated alcohols to the active methylene group. Part I. The action of ethyl acetoacetate on linalool and geraniol". J. Chem. Soc. 1940, 704-706) in the presence of base and with high reaction temperature takes place through an intermediate enol which then rearranges in an electrocyclic Claisen rearrangement. The follow up is a decarboxylation. With palladium(0) as a catalyst, the reaction (Tsuji, Tetrahedron Letters, Volume 21, Issue 33, 1980, Pages 3199-3202) is much milder with an intermediate allyl cation / carboxylic acid anion organometallic complex.

A GGA-like compound of the invention finds is use among others in various therapeutic applications, including those known for GGA. Exemplary uses are prophylactic and/or therapeutic treatment of gastric ulcers, ischemic brain injury, endotoxin shock, atrial fibrillation, glaucoma, hepatitis C, dermatosis, ischemia/reperfusion disorders, injury, inflammation, infection and organ transplants, or as a topical anti-aging active substance.
In one aspect, the compound is used to induce a heat shock protein. Heat shock proteins (HSPs) function as molecular chaperones to prevent protein aggregation and facilitate the folding of non-native proteins, particularly new peptides emerging from ribosomes. Molecular chaperones recognize non-native proteins, predominantly via exposed hydrophobic residues, and bind selectively to those proteins to form relatively stable complexes. In these complexes, the protein is protected and able to fold into its native form. HSPs show increased levels of expression when cells are subjected to elevated temperatures and other metabolic stresses. Examples of metabolic stresses which elicit elevated expression of heat shock proteins include: decreased glucose availability; increased intercellular calcium levels; and decreased blood flow.

For instance, a compound of the invention is advantageously used in the treatment and/or prevention of a disease which is associated with the accumulation of misfolded proteins, for example supraventricular arrythmia, preferably atrial fibrillation (AF). Repetitive, forceful muscular contractions, i.e. physical exercise, cause changes in the expression patterns of genes and proteins. These changes can result in muscle adaptations such as muscle atrophy via muscle protein catabolism or muscle hypertrophy via muscle protein accretion. During hypertrophy, numerous nascent proteins are formed. An increase in the presence of molecular chaperones, like HSPs, will act to enhance the stability of these nascent proteins until they can fold into their native forms. Thus, compounds disclosed herein also find their use in situations of enhanced protein turnover, such as the environment in muscle following exercise, it would be advantageous for an individual to have a means of increasing the stability of rapidly forming proteins in order to reduce the catabolism of these new non-native state proteins.

In one aspect, the invention provides the use of a compound of the general formula I herein above
wherein
Y is selected from the group consisting of 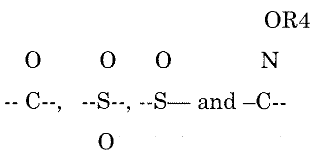 wherein R4 is hydrogen or a lower alkyl group
R1 is a lower alkyl group, optionally substituted with 1 to 4 substituents selected from the group consisting of halogen, hydroxyl; lower alkyl; lower alkoxy; halogenated lower alkyl; halogenated lower alkoxy; cyano; a 5- or 6-membered (hetero)aromatic ring which may be substituted by hydroxyl, lower alkyl, lower alkoxy, halogen, amino, lower alkylamino, cyano, nitro or other (substituted) (hetero)aromatic rings
R2 is hydrogen, lower alkyl or substituted lower alkyl group
R3 is a saturated or unsaturated aliphatic moiety of 1-15 carbon atoms, preferably Cl-C10, optionally substituted with 1 to 5 substituents selected from the group consisting of halogen, hydroxyl, lower alkyl, lower alkoxy, hydroxyl-lower alkyl, halogen, amino, lower alkylamino and cyano nitro, a 5-or 6-membered hetero)aromatic ring which may be substituted by hydroxyl, lower alkyl, lower alkoxy, halogen, amino, lower alkylamino; cyano, nitro or other (substituted) (hetero)aromatic rings
or a pharmaceutically acceptable salt thereof,
under the proviso that the compound is not geranylgeranylacetone (GGA; IUPAC name: (5E, 9E,13E)-6,10,14,18-tetramethylnonadeca-5, 9,13,17-tetraen-2-one) for the induction of a heat shock protein, for example in the treatment and/or prophylaxis of a disease which is associated with the accumulation of misfolded proteins.

The compound according to this invention can be administered orally in a form of powder, tablet, granule, capsule, pill and liquid, and parenterally in injection, suppository and the like. The dose amounts of 50-2000 mg a day when used to treat an adult. It is desirable that the dose varies properly depending upon the symptoms and the administration is divided by proper intervals.

The compound according to this invention can be prepared for administration by any conventional process for pharmaceutical preparation. Therefore, this invention provides the pharmaceutical preparations suitable for a medicine for the human body, which comprises at least one of the compounds according to this invention. Such preparations are provided to be administered by a conventional method with any required carrier or excipient for the production of medicine.

This preparation is desirably provided in a suitable form for absorption from the alimentary canal. The tablet and capsule for oral administration are a form of unit dose, and may contain conventional excipients such as a binder, for example, syrup, gum arabic, gelatine, sorbite, tragacanth gum or polyvinyl pyrolidone; a constituent, for example, lactose, corn starch, calcium phosphate, sorbite of glycine; a lubricant, for example, magnesium stearate, talc, polyethylene glycol or silica; a disintegrator, for example, potato starch; and an acceptable wetting agent, for example, sodium lauryl sulfate. The tablet may be coated by a well-known method in the art. The liquid preparations for oral administration may be an aqueous or oily suspension, solution, syrup, elixir and the like. Alternatively, they may be a dry product which is re-dissolved in water or other suitable vehicle prior to use. Such liquid preparations may contain conventional additives, for example, a suspending agent, such as sorbite syrup, methyl cellulose, glucose/sugar syrup, gelatine, hydroxyethyl cellulose, carboxymethyl cellulose, aluminium stearate gel and hydrogenated edible fat; an emulsifier such as lecithine, sorbitane mono-oleate and gum arabic; a nonaqueous vehicle such as almond oil, fractionated coconut oil, oiliness ester, propylene glycol and ethyl alcohol; an antiseptics such as methyl p-hydroxy benzoate, propyl p-hydroxybenzoate and sorbic acid.

The preparation for injection are provided in a unit-dose ampoule or vial with an additive antiseptics. The preparations may be a form of suspension, solution or emulsion in an oily or aqueous vehicle, and also may contain a formulating agent such as a suspension agent, stabilizer and/or dispersant. On the other hand, the active ingredient may be a form of powder which is re-dissolved in a suitable vehicle, for example, sterilized water free from exothermic materials, prior to use.

Also encompassed is a nutritional, neutraceutical or cosmetic composition or supplement comprising a compound according to the invention. The composition of the present invention may be administered in a dosage form having controlled release characteristics, e.g. time-release. Furthermore, the controlled release may be in forms such as a delayed release of active constituents, gradual release of active constituents, or prolonged release of active constituents. Such active constituents release strategies extend the period of bioavailability or target a specific time window for optimal bioavailability. Advantageously the composition may be administered in the form of a multi-compartment capsule which combines both immediate release and time-release characteristics. Individual components of the composition may be contained in differential compartments of such a capsule such that the specific components may be released rapidly while others are time-dependently released. Alternatively, a uniform mixture of the various components of the present invention may be divided into both immediate release and time-release compartments to provide a multi-phasic release profile.

According to various embodiments of the present invention, the supplement may be consumed in any form. For instance, the dosage form of the nutritional supplement may be provided as, e.g., a powder beverage mix, a liquid beverage, a ready-to-eat bar or drink product, a capsule, a liquid capsule, a softgel capsule, a tablet, a caplet, or as a dietary gel. The preferred dosage form of the present invention is as a softgel capsule. As used herein, a serving of the present nutritional composition typically comprises from about 1 mg to about 300 mg of a GGA bioisoster of the invention. More preferably, a serving of the present nutritional composition comprises from about 25 mg to about 150 mg, most preferably comprises from about 25 mg to about 75 mg of a GGA-lie compound disclosed herein.
Additionally, the nutritional supplement set forth in the example embodiment herein may further contain any appropriate number and type of excipients, as is well known in the art.

The present composition or those similarly envisioned by one of skill in the art, may be utilized in methods to enhance the expression of heat shock proteins in cells.

### LEGEND TO THE FIGURE

Figure 1: Structures and IUPAC names of exemplary GGA-like compounds of the invention.

### EXAMPLES

### Example 1: Synthesis of analogues /bioisosters of geranylgeranylacetone (route 1). In this Example, a compound wherein R3 = geranylgeranyl and Y is C=O (carbonyl) is taken as an example.

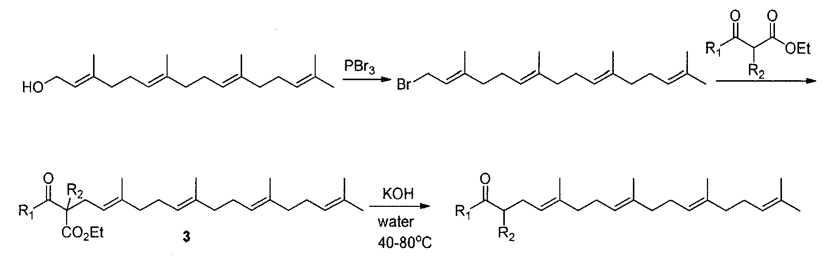

### Example 2: Synthesis of analogues /bioisosters of geranylgeranylacetone (route 2, by a Carroll rearrangement)

In this Example, a compound wherein R3 = geranylgeranyl and Y is C=O (carbonyl) is taken as an example.

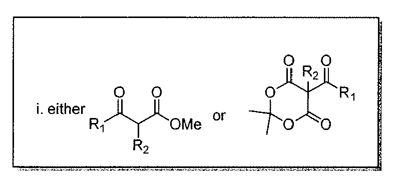

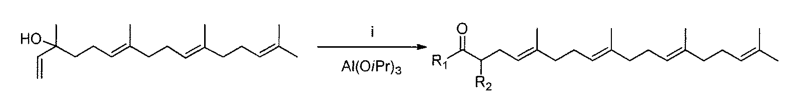

### Example 3: Synthesis of analogues/bioisosters of geranylgeranylacetone wherein Y is N=OR₄ via reaction of geranylgeranylacetone with (0-alkyl) hydroxylamines.

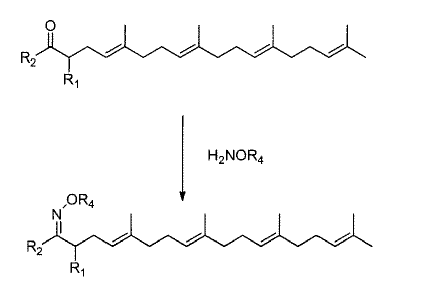

### Example 4: Synthesis of analogues/bioisosters of geranylgeranylacetone (sulfones/sulfoxides) wherein Y is SO or S0₂.

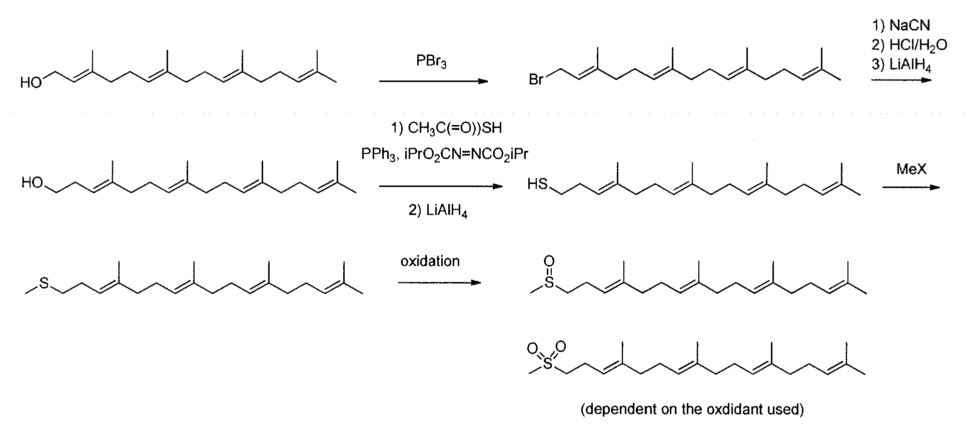

## Claims

1. A compound of the general formula I 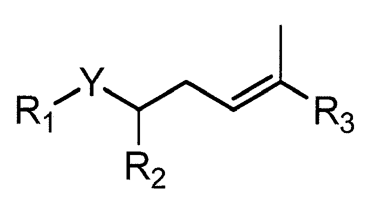 wherein
Y is selected from the group consisting of 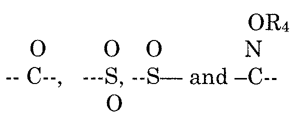 wherein R4 is hydrogen or a lower (e.g. C1-C4) alkyl group
R1 is a lower alkyl group substituted with 1 to 4 substituents selected from the group consisting of halogen, hydroxyl; lower alkyl; lower alkoxy; halogenated lower alkyl; halogenated lower alkoxy; cyano; a 5- or 6-membered (hetero)aromatic ring which may be substituted by hydroxyl, lower alkyl, lower alkoxy, halogen, amino, lower alkylamino; cyano, nitro; and other (substituted) (hetero)aromatic rings;
R2 is hydrogen, lower alkyl or substituted lower alkyl group, preferably C1-C10 alkyl, more preferably C1-C5 alkyl;
R3 is a saturated or unsaturated aliphatic moiety of 1-15 carbon atoms, preferably C1-C10, optionally substituted with 1 to 5 substituents selected from the group consisting of halogen, hydroxyl, lower alkyl, lower alkoxy, hydroxyl-lower alkyl, halogen, amino, lower alkylamino and cyano, nitro, a 5-or 6-membered hetero)aromatic ring which may be substituted by hydroxyl, lower alkyl, lower alkoxy, halogen, amino, lower alkylamino; cyano, nitro or other (substituted) (hetero)aromatic rings;
or a pharmaceutically acceptable salt thereof.

2. A compound of the general formula I, wherein
Y is

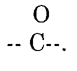

3. A compound of the general formula I 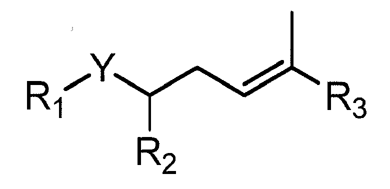 wherein
Y is selected from the group consisting of 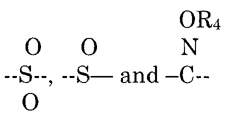 wherein R4 is hydrogen or a lower (e.g. C1-C4) alkyl group
R1 is a lower alkyl group substituted with 1 to 4 substituents selected from the group consisting of halogen, hydroxyl; lower alkyl; lower alkoxy; halogenated lower alkyl; halogenated lower alkoxy; cyano; a 5- or 6-membered (hetero)aromatic ring which may be substituted by hydroxyl, lower alkyl, lower alkoxy, halogen, amino, lower alkylamino; cyano, nitro; and other (substituted) (hetero)aromatic rings;
R2 is hydrogen, lower alkyl or substituted lower alkyl group, preferably C1-C10 alkyl, more preferably C1-C5 alkyl.
R3 is a saturated or unsaturated aliphatic moiety of 1-15 carbon atoms, preferably C1-C10, optionally substituted with 1 to 5 substituents selected from the group consisting of halogen, hydroxyl, lower alkyl, lower alkoxy, hydroxyl-lower alkyl, halogen, amino, lower alkylamino and cyano, nitro, a 5-or 6-membered hetero)aromatic ring which may be substituted by hydroxyl, lower alkyl, lower alkoxy, halogen, amino, lower alkylamino; cyano, nitro or other (substituted) (hetero)aromatic ring;
or a pharmaceutically acceptable salt thereof.

4. Compound according to any one of claims 1-3, wherein R3 is based on a terpene, preferably wherein R3 is a geranylgeranyl (1), farnesyl (2) or geranyl (3) moiety.

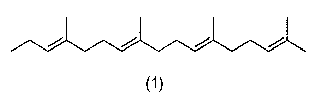

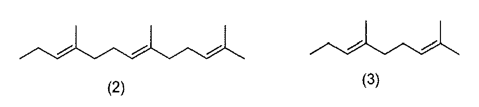

5. Compound according to any one of claims 1-4, wherein R4 is hydrogen.

6. Compound selected from Figure 1.

7. Compound according to any of the preceding claims, for use as a therapeutic substance.

8. Compound according to any one of claims 1-6, for the treatment and/or prevention of a disease which are associated with the accumulation of misfolded proteins.

9. Pharmaceutical composition comprising a compound according to any one of claims 1-6 and a pharmaceutically acceptable carrier and/or adjuvant.

10. The use of a compound according to any one claims 1-6 for the preparation of a medicament for the treatment and/or prophylaxis of a disease which is associated with the accumulation of misfolded proteins.

11. Use according to claim 10, wherein the disease is supraventricular arrythmia, preferably atrial fibrillation (AF).

12. Compound of the general formula I 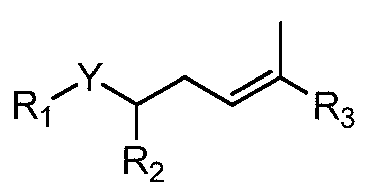 wherein
Y is selected from the group consisting of 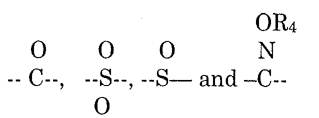 wherein R4 is hydrogen or a lower alkyl group
R1 is a lower alkyl group, optionally substituted with 1 to 4 substituents as defined for R1 in claim 1;;
R2 is hydrogen, lower alkyl or substituted lower alkyl group
R3 is a saturated or unsaturated aliphatic moiety of 1-15 carbon atoms, preferably Cl-C10, optionally substituted with 1 to 5 substituents as defined for R3 in claim 1,
or a pharmaceutically acceptable salt thereof,
under the proviso that the compound is not geranylgeranylacetone (IUPAC name: (5E,9E,13E)-6,10,14,18-tetramethylnonadeca-5,9,13,17-tetraen-2-one)
for the treatment and/or prophylaxis of a disease which is associated with the accumulation of misfolded proteins.

13. Compound according to claim 12, wherein the disease is supraventricular arrythmia, preferably atrial fibrillation (AF).

14. A method for providing a compound according to any one of claims 1-6, comprising a Carroll rearrangement of a vinyl B-alcohol with (substituted) alkyl acetoacetates, diketenes or acylated Meldrum's acids.
